# EUROPEAN PATENT SPECIFICATION

(11) **EP 3 304 370 B1**
(45) Date of publication and mention of the grant of the patent: **30.12.2020**
(21) Application number: 16724732.9
(22) Date of filing: 16.05.2016
(51) Int. Cl.: G16H 40/40

(54) **INFUSION PUMP SYSTEM AND METHOD WITH MULTIPLE DRUG LIBRARY EDITOR SOURCE CAPABILITY**
INFUSIONSPUMPENSYSTEM UND VERFAHREN MIT MEHRFACHEN WIRKSTOFFDATENBANKEDITORQUELLEN
PROCÉDÉ ET SYSTÈME DE POMPE À PERFUSION POUVANT UTILISER UN ÉDITEUR DE PHARMACOTHÈQUE À SOURCE MULTIPLE

(30) Priority: 26.05.2015 US 201562166381 P
(43) Date of publication of application: 11.04.2018
(73) Proprietor: ICU Medical, Inc., San Clemente, CA 92673 (US)
(72) Inventor: JACOBSON, James D., Lindenhurst, Illinois 60046 (US); BELKIN, Anatoly S., Glenview, Illinois 60026 (US)
(74) Representative: Invent Horizon IP
(86) International application number: PCT/IB2016/052822
(87) International publication number: WO 2016/189417

(56) References cited:
- WO-A2-2014/100736
- WO-A2-2014/131730
- US-A1- 2008 091 466

## Description

### TECHNICAL FIELD

The present invention relates to medical devices. More specifically, the invention relates to infusion pump systems and methods with multiple drug library editor source capability.

### BACKGROUND OF THE INVENTION

Infusion pumps are medical devices that deliver fluids, including nutrients and medications such as antibiotics, chemotherapy drugs, and pain relievers, into a patient's body in controlled amounts. Many types of pumps, including large volume, patient-controlled analgesia (PCA), elastomeric, syringe, enteral, and insulin pumps, are used worldwide in healthcare facilities, such as hospitals, and in the home. Clinicians and patients rely on pumps for safe and accurate administration of fluids and medications.

More than 90 percent of surgical patients and one-third of non-surgical patients receive some form of intravenous therapy while in the hospital. Industry reports indicate an estimated 400,000 drug related injuries occur in hospitals annually generating $3.5 billion in extra medical costs. Safety software, including drug error reduction systems (DERS), has been developed to reduce medication errors, enhance quality care, and improve workflow.

Drug error reduction systems (DERS) typically use a drug library editor (DLE) to develop drug libraries including protocols, rule sets, and/or pump configuration settings, which are then loaded onto an infusion pump. The drug library at the infusion pump provides medication pick lists for the caregiver to select the desired therapy, medication profiles including hard and soft limits, and pump configuration settings including but not limited to distal pressure occlusion limits, air-in-line limits, callback settings, backlight display settings, etc..

Drug library editors are currently one of two types: dedicated DLEs or enterprise DLEs. Dedicated DLEs can only be connected to one infusion pump at a time to download the drug library to the individual infusion pump. Enterprise DLEs are part of an integrated healthcare facility system and can be connected to download a drug library to a number of infusion pumps.

Dedicated DLEs for a single type of infusion pump can implement drug error reduction systems in a simple cost-effective manner, but typically have reduced functionality when compared to enterprise DLEs. Dedicated DLEs generally provide protocol based libraries, in which the point of care caregiver selects a predefined protocol from a list, then accepts or modifies the predefined infusion parameters for the infusion to be administered. Dedicated DLEs work well for home, nursing homes, and other smaller facilities in which a limited number of medications are used.

Enterprise DLEs for multiple infusion pumps can implement drug error reduction systems as part of more complex and expensive systems having large drug libraries, rich functionality, and sophisticated reporting tools. Enterprise DLEs can be used to provide drug profile-based libraries, with the point of care caregiver selecting a medication from a medication list, then programming the infusion to be administered under limits assigned to the medication. Enterprise DLEs work well for hospitals, treatment centers, surgery centers, and other large facilities.

Unfortunately, no infusion pump is presently available which is able to obtain drug libraries from different types of drug library editors, e.g., from both dedicated DLEs and enterprise DLEs. Although in the case of enterprise DLEs, one DLE may be used with different types of infusion pumps, no infusion pump can receive drug libraries from more than one DLE even though one infusion pump type can be used in different settings, such as the home or hospital. In present practice, one infusion pump can receive a drug library from one specified type of drug library editor, but no other. For example, one infusion pump can receive a drug library from an enterprise drug library editor, but not a dedicated drug library editor. Similarly, a different kind of infusion pump can receive a drug library from a dedicated drug library editor, but not an enterprise drug library editor. Thus, the manufacturer must produce and the users must select different infusion pumps and associated DLEs for different applications, increasing cost and complexity. Different programming is required for dedicated DLEs and enterprise DLEs, increasing the confusion and effort required for programmers of different DLEs. Healthcare systems which cover multiple levels of care from home care to hospital care must maintain different DLEs for different levels, increasing costs to the healthcare system and to the consumers.

US 2008/091466A1 discloses a drug library editor as part of a medication management unit (MMU). It is disclosed that MMUs can be used to configure medical devices, such as medication delivery pumps, as well as store activity information regarding the activity of each of the medical devices. Drug library editors can be implemented in a remote environment.

WO 2014/100736A2 discloses that a user can load drug administration library (DAL) files to medical devices by using an editor service to first load the files to a storage and then transferring the files from the storage to the medical devices. It is disclosed that at least one editor computer may be configured to communicate with a server via a network in a client-server based model such that an editor computer is able to access a drug library editing software.

WO 2014/131730 A2 discloses that operational data, which is stored on a drug library server, can be shared among medical devices in a network, and that the drug libraries can be edited. It is also disclosed that when drug libraries are installed locally on the medical devices, it is cumbersome to distribute new or updated drug libraries throughout an entire healthcare environment without having duplicate variants of drug libraries being installed on different medical devices.

It would be desirable to have infusion pump systems and methods with multiple drug library editor source capability that would overcome the above disadvantages.

### SUMMARY OF THE INVENTION

One aspect of the present invention provides an infusion pump system including a first drug library editor operable to generate a first drug library; a second drug library editor operable to generate a second drug library; and an infusion pump operable to connect to either one of the first drug library editor and the second drug library editor, the infusion pump having an operational drug library being one of the first drug library received from the first drug library editor and the second drug library received from the second drug library editor. The first drug library editor is one of a dedicated drug library editor and an enterprise drug library editor, and the second drug library editor is the other of the one of the dedicated drug library editor and the enterprise drug library editor.

Another aspect of the present invention provides a method of configuring one or more infusion pumps including providing an infusion pump having an operational drug library; preparing a first drug library on a first drug library editor; loading the first drug library into the infusion pump from the first drug library editor as the operational drug library; delivering an infusion from the infusion pump in accordance with the first drug library; preparing a second drug library on a second drug library editor; loading the second drug library into the infusion pump from the second drug library editor as the operational drug library; and delivering an infusion from the infusion pump in accordance with the second drug library. The first drug library editor is one of a dedicated drug library editor and an enterprise drug library editor, and the second drug library editor is the other of the one of the dedicated drug library editor and the enterprise drug library editor.

Another aspect of the present invention provides a method of configuring one or more infusion pumps including providing an infusion pump adapted to receive a drug library from one of multiple drug library editors and having an operational drug library; preparing one of a first drug library on a first drug library editor or a second drug library on a second drug library editor; selecting one of the first drug library editor and the second drug library editor as a source for a received drug library; loading the received drug library into the infusion pump as the operational drug library; and delivering an infusion from the infusion pump in accordance with the operational drug library, wherein the first drug library editor is one of a dedicated drug library editor and an enterprise drug library editor, and the second drug library editor is the other of the one of the dedicated drug library editor and the enterprise drug library editor.

Yet another aspect of the present invention provides an infusion pump for selectable use with either one of a first drug library editor operable to generate a first drug library and a second drug library editor operable to generate a second drug library, the first drug library editor being one of a dedicated drug library editor and an enterprise drug library editor, and the second drug library editor being the other of the one of the dedicated drug library editor and the enterprise drug library editor. The infusion pump includes a memory operable to store an operational drug library; a flow controller operably connected to the memory; and a fluid driver operably connected to the flow controller. The memory is operable to receive a selection of one of the first drug library from the first drug library editor and the second drug library from the second drug library editor as the operational drug library, and the flow controller is operable to control the fluid driver in accordance with the received operational drug library.

The foregoing and other features and advantages of the invention will become further apparent from the following detailed description of the presently preferred embodiments, read in conjunction with the accompanying drawings. The detailed description and drawings are merely illustrative of the invention rather than limiting, the scope of the invention being defined by the appended claims and equivalents thereof.

### BRIEF DESCRIPTION OF THE DRAWINGS

**FIG. 1** is a block diagram of an infusion pump system with multiple drug library editor source capability in accordance with the present invention;
**FIG. 2** is a block diagram of a medication management system with an enterprise drug library editor for an infusion pump system with multiple drug library editor source capability in accordance with the present invention.
**FIG. 3** is a block diagram of a dedicated infusion pump system with a dedicated drug library editor for an infusion pump system with multiple drug library editor source capability in accordance with the present invention.
**FIG. 4** is a block diagram of an infusion pump with multiple drug library editor source capability in accordance with the present invention;
**FIG. 5** is a flowchart of a method of use for an infusion pump system with multiple drug library editor source capability in accordance with the present invention.
**FIG. 6** is a flowchart of another method of use for an infusion pump system with multiple drug library editor source capability in accordance with the present invention.

Like elements share like reference numbers throughout the various figures.

### DETAILED DESCRIPTION OF PRESENTLY PREFERRED EMBODIMENTS

An infusion pump system with multiple drug library editor source capability is described herein. The infusion pump can receive an operational drug library from either one of a dedicated drug library editor or an enterprise drug library editor. Thus, the infusion pump can be used with a medication management system such as used in a large enterprise medical care facility like a hospital or in a small dedicated care facility such as a nursing home or the home of a patient.

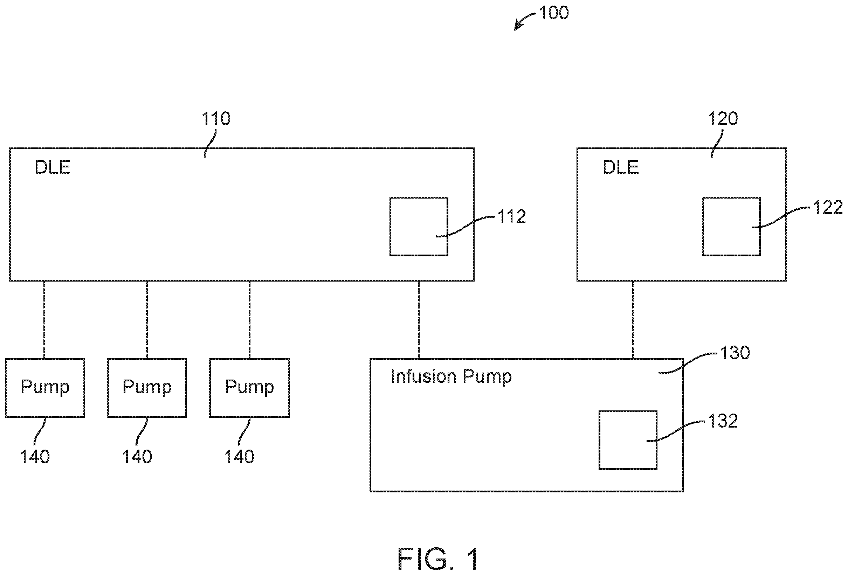

**FIG. 1** is a block diagram of an infusion pump system with multiple drug library editor source capability in accordance with the present invention. In this example, the infusion pump system includes a dedicated drug library editor and an enterprise drug library editor. The infusion pump provides a therapeutic fluid from a fluid reservoir to a patient in accordance with operating parameters and/or limits established in an operational drug library.

An infusion pump system **100** includes a first drug library editor (DLE) **110,** a second drug library editor (DLE) **120,** and an infusion pump **130.** The first drug library editor **110** is operable to generate a first drug library **112** and the second drug library editor **120** is operable to generate a second drug library **122.** The infusion pump **130** is operable to connect to either one of the first drug library editor **120** or the second drug library editor **130** as desired for a particular application. The infusion pump **130** has an operational drug library **132,** which is one of the first drug library **112** received from the first drug library editor **110** or the second drug library **122** received from the second drug library editor **120.** The first drug library editor **110** is one of a dedicated drug library editor and an enterprise drug library editor, and the second drug library editor **120** is the other of the one of the dedicated drug library editor and the enterprise drug library editor. Thus, one drug library editor is of one type and the other drug library editor is of a different type.

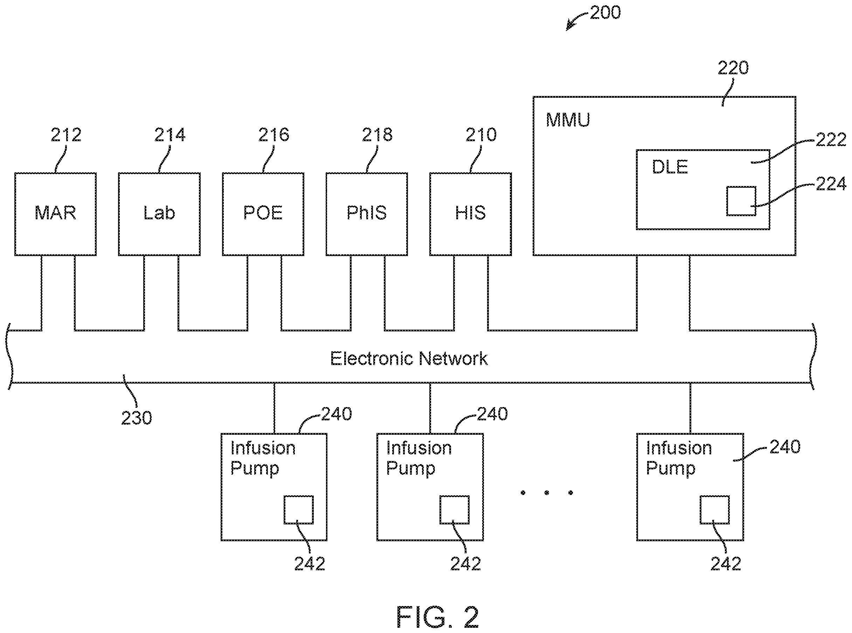

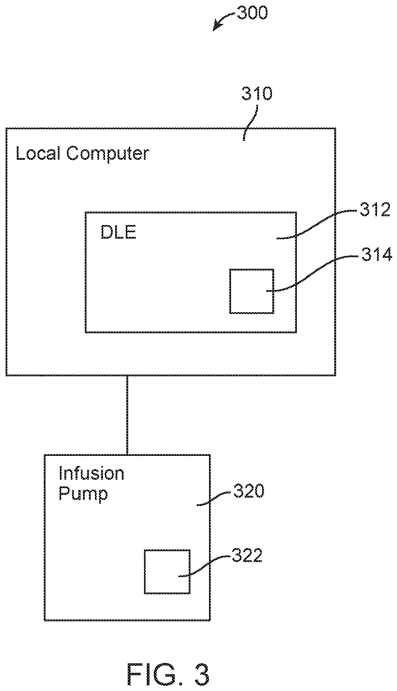

In this example, the first drug library editor **110** is the enterprise drug library editor and the second drug library editor **120** is the dedicated drug library editor. The first drug library editor **110** can provide operational drug libraries to a number of infusion pumps **140** in addition to the infusion pump **130.** The enterprise drug library editor **110** is part of a medication management unit **220** that is in electronic communication or networked with other electronic information systems within a healthcare system, which facilitates or provides bi-directional communication between the pumps and the other electronic information systems, as best seen in **FIG. 2****.** The second drug library editor **120** only provides drug libraries to one infusion pump at a time, in this case infusion pump **130.** The dedicated drug library editor **120** as defined herein is a standalone drug library editor that is not networked to other information systems within a healthcare system. The dedicated drug library editor is operably connectable to and provides a drug library for a single type of infusion pump, typically a limited set of local infusion pumps such as one to fifteen infusion pumps in a single geographic location. The dedicated drug library editor is also limited in the functions it performs and typically only handles the creation, editing and downloading of a drug library to the pump. The enterprise drug library editor as defined herein is a drug library editor operably connectable to and providing drug libraries for a number of different types of infusion pumps, for example a multitude (more than fifteen) geographically dispersed pumps of various types (PCA, syringe, large volume parenteral, etc.). The enterprise drug library editor is typically a part of a larger suite of software applications run on or connected through a client computer to a server that has more functions such as bi-directional communications between the pump and server, as well as sophisticated logging and reporting on pump programming, user input, and operation (including but not limited to alarms, alerts, messages, overrides, program modifications, etc.). The application of the dedicated drug library editor and the enterprise drug library editor are described below in association with **FIGS. 2** **&** **3****,** respectively.

Referring to **FIG. 1****,** the first drug library editor **110** and second drug library editor **120** are operably connectable to the infusion pump **130** as indicated by the dashed lines between the drug library editors **110, 120** and the infusion pump **130.** The connections between the drug library editors **110, 120** and the infusion pump **130** can be wired, wireless, or a combination of wired and wireless. For the enterprise drug library editor, the connections between the drug library editors **110, 120** and the infusion pump **130** can include intermediate components as desired for a particular application. One of the drug library editors **110, 120** can be connected to the infusion pump **130** to transfer the drug library **112, 122,** respectively, to the infusion pump **130** as the operational drug library **132,** and then the connection between the drug library editor and the infusion pump can be disconnected.

The first drug library editor **110** and second drug library editors **120** are used to create and/or modify the first drug library **112** and the second drug library **122,** respectively. The drug library editor operates on the drug library within the drug library editor to support, maintain, edit, and/or export the operational drug library received by the infusion pump. A programmer (not shown), such as a licensed pharmacist, doctor, biomedical engineer, or the like, working with the drug library editor on a user interface can create and/or modify the drug libraries as desired for a particular application. The drug library editor can be deployed in a medication management system on one or more computers and enables the programmer to import, export, and edit whole drug libraries and individual drug library values to control and customize a drug library according to hospital preferences. In one example, drug libraries in the drug library editor can be formulated and edited using a conventional database management software platform, such as SQL Server or SQL Desktop Engine by Microsoft of Redmond, Washington.

The first drug library **112** and the second drug library **122** can each be part of a customizable drug library database available to the first drug library editor **110** and second drug library editors **120,** respectively. The drug library includes drug and infusion pump related information, which may include but is not limited to drug name, drug class, drug concentration, drug amount, drug units, diluent amount, diluent units, dosing units, delivery dose or rate, medication parameters or limits (hard or soft), device/infuser settings and/or modes, clinical care area (CCA) designations and constraints, library version, and the like. The drug library can also include operating parameters for reporting from each of the infusion pumps back to the medication management system.

**FIG. 2** is a block diagram of a medication management system **200** with an enterprise drug library editor for an infusion pump system with multiple drug library editor source capability in accordance with the present invention. The enterprise drug library editor is a drug library editor operably connectable to and providing drug libraries for a multitude of infusion pumps, for example more than fifteen pumps. One example of an enterprise drug library editor **222** is the Hospira MedNet™ Meds™ drug library editor from Hospira, Inc. of Lake Forest, IL, U.S.A. The infusion pumps served by the enterprise drug library editor are typically of various different types. Types in this context can mean different operating mechanisms, manufacturers, models, or software or hardware versions, by way of example and not limitation.

The medication management system **200** provides an integrated enterprise-wide medication management system that reduces the risks of medication error and improves patient safety. The medication management system (MMS) **200** includes a hospital information system (HIS) **210,** a medication management unit (MMU) **220,** an electronic network **230,** and infusion pumps **240.** In one embodiment, the medication management system (MMS) **200** operates in a hospital environment which can be construed broadly as used herein to mean any medical care facility, including but not limited to a hospital, treatment center, clinic, doctor's office, day surgery center, hospice, nursing home, and any of the above associated with a home care environment. In one embodiment, the medication management system **200** operates in a large medical care facility, such as a hospital or treatment center. The medication management unit **220** deployed on one or more enterprise servers includes a drug library editor **222** with a drug library **224.** Each of the infusion pumps **240** includes an operational drug library **242,** with each of the operational drug libraries **242** for each individual infusion pump **240** being tailored for the patient using each individual infusion pump **240,** i.e., the operational drug libraries **242** can be different from each other as required to meet the therapy needs of a particular patient, the intended use of the infusion pump, or the location of the pump or patient. The medication management system **200** can include one or more computers/servers with associated software as required for a particular application.

The medication management system (MMS) **200** can optionally include additional information systems in communication with the medication management unit (MMU) **220** and infusion pumps **240** across the electronic network **230.** In various embodiments, the medication management system (MMS) **200** can optionally include a pharmacy information system (PhIS) **218** for delivering drug orders to a hospital information system (HIS) **210,** a physician/provider order entry (POE) **216** permitting a healthcare provider to deliver a medication order prescribed for a patient to the hospital information system, a lab system **214** to monitor patient data and to deliver updated patient-specific information such as lab tests, measurements or the like to the MMU **220,** and/or a medication administration record system (MAR) **212** for maintaining medication records. The medication management system (MMS) **200** can also include monitoring devices (not shown) in communication with the MMU **220** and/or lab system **214,** for monitoring condition of the patient using each of the infusion pumps **240.**

The hospital information system (HIS) **210** can be any information system operable to communicate across the hospital environment. The hospital information system **210** can include and/or communicate with the medication administration record system (MAR) **212** to maintain medication records and the pharmacy information system (PhIS) **218** to deliver drug orders to the hospital information system **210.** The physician/provider order entry (POE) device **216** can permit a healthcare provider to deliver a medication order prescribed for a patient to the hospital information system **210** directly or indirectly via the PhIS **218.** One skilled in the art will also appreciate that a medication order can be sent to the MMU **220** directly from the PhIS **218** or POE device **216.** As used herein the term medication order is defined as an order or "prescription" to administer something that has a physiological impact on a person or animal, including but not limited to liquid or gaseous fluids, drugs or medicines, liquid nutritional products and combinations thereof.

The medication management unit **220** includes the drug library editor **222** with a drug library **224.** The drug library editor **222** operates on the drug library **224** within the drug library editor **222** to support, maintain, edit, import and/or export the operational drug library received by the infusion pumps **240.** A programmer (not shown), such as a licensed pharmacist, doctor, or the like, can work with the drug library editor on a user interface to create and/or modify the drug library **224** as desired for a particular application. The drug library editor **222** can be deployed in the medication management unit **220** on one or more computers and enables the programmer to import, export, and edit whole drug libraries and individual drug library values to control and customize a drug library according to hospital preferences.

The medication management unit **220** can perform other functions in addition to being the drug library editor. In one embodiment, the medication management unit **220** can further maintain patient safety by reviewing medication orders, drug-drug compatibility, and medication delivery time sequences. In another embodiment, the medication management unit **220** can modulate performance of a medication order based on laboratory data or other newly received patient information. In another embodiment, the medication management unit **220** can monitor the status of the infusion pumps and infusion status progress (including alarms, event logs, and pump user interface inputs), generate reports, and control software or operating code updates to the infusion pumps.

The electronic network **230** can be any information network operable to connect the hospital information system (HIS) **210,** medication management unit (MMU) **220,** and infusion pumps **240.** The electronic network **230** can be any combination of wired and/or wireless networks as desired for a particular application. The electronic network **230** can provide communication within a single location, such as a single hospital, or can provide communication over various geographic locations, such as a healthcare network.

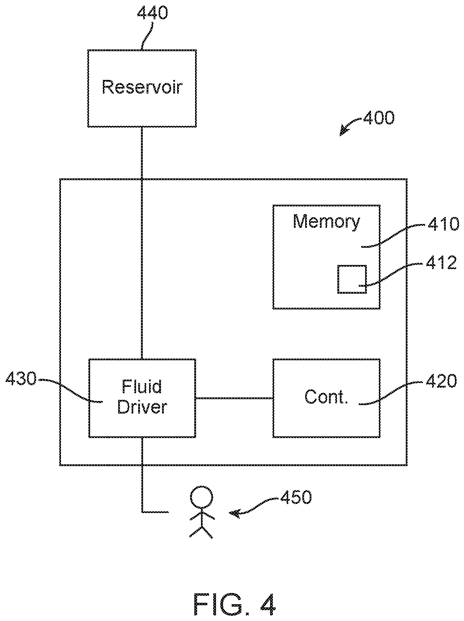

The infusion pumps **240** include one or more infusion pumps with multiple drug library editor source capability. The infusion pumps **240** can be any type of pump, including but not limited to a pump having a pumping mechanism or fluid driver, which acts upon a cassette, reservoir, vial, syringe, or tubing to convey medication or fluid to or from a patient. Exemplary pumps include but are not limited to enteral pumps, infusion pumps, patient controlled analgesia (PCA) or pain management medication pumps, syringe pumps, peristaltic pumps and the like. The pumps could be stationary (pole or bedside mounted and plugged into an AC outlet) or ambulatory (worn or carried by the patient and power by one or more batteries) or some combination of these types or individual features. In one embodiment, the infusion pump with multiple drug library editor source capability can be an infusion pump as described below in association with **FIG. 4****.**

The Hospira MedNet™ safety software available from Hospira, Inc., of Lake Forest, Illinois, U.S.A. is one example of software that resides in the medication management unit or MMU **220** of a medication management system **200.** The Hospira MedNet™ safety software is described in US Patent Application Serial Number 10/930,358 filed August 31, 2004, entitled Medication Management System (now US Patent Number 7,895,053, issued February 22, 2011). Hospira MedNet™ safety software links infusion devices with the hospital pharmacy and hospital information systems, enabling clinicians to better bi-directionally communicate, understand and manage IV infusion information with respect to infusion pumps at the point of care. As stated above, one example of an enterprise drug library editor is Hospira MedNet™ Meds™ software. An early version of such software is described in U.S. Pat. No. 8,065,161 to Howard et al., entitled System for Maintaining Drug Information and Communicating with Medication Delivery I Devices. Using such a drug library editor, hospital pharmacists work in collaboration with a cross-functional medical team to develop customized drug libraries and dose recommendations, which are then programmed into a database and transferred to the infusion pump. The drug libraries can incorporate both hard and soft dose limits, and can customize clinical decision rules for multiple clinical care areas (CCAs). In various applications, the Hospira MedNet™ safety software and Hospira MedNet™ Meds™ drug library editor software can be used with the Hospira Plum A+™ general infusion system, the Hospira Plum A+™ 3 triple-channel device, the Hospira LifeCare PCA™ patient-controlled analgesia system, or the Hospira Symbiq™ infusion pump.

**FIG. 3** is a block diagram of an infusion pump system with a standalone, dedicated drug library editor for an infusion pump system with multiple drug library editor source capability in accordance with the present invention. The dedicated drug library editor is a drug library editor operably connectable to and providing a drug library for a single type of infusion pump or a limited set of from one to fifteen infusion pumps of the same type.

The infusion pump system **300** includes a local computer **310** and an infusion pump **320.** A dedicated drug library editor **312** having a drug library **314** is deployed on the local computer **310.** The infusion pump **320** includes an operational drug library **322.** In one embodiment, both the local computer **310** and infusion pump **320** are located at the point of care (POC) of the patient, such as a hospice, nursing home, home care environment, or the like. In another embodiment, the local computer **310** is located in a non-patient care area such as a biomedical engineering area and is used to setup or configure infusion pumps for distribution and use in other locations. In one embodiment, the infusion pump system **300** can provide various exemplary therapies, such as continuous therapy, multistep therapy, intermittent therapy, total parenteral nutrition (TPN), Patient Controlled Analgesia (PCA) therapy, epidural therapy, or the like.

The local computer **310** can be any computing device capable of deploying the dedicated drug library editor **312** as desired for a particular application. Exemplary computing devices include personal computers, tablet computers, personal digital assistants, handheld computers, and the like. The local computer **310** can communicate with the infusion pump **320** when connected to the infusion pump **320** over wired or wireless connections.

The infusion pump **320** is an infusion pump with multiple drug library editor source capability. The infusion pumps **320** can be any type of pump, including but not limited to a pump having a pumping mechanism or fluid driver, which acts upon a cassette, reservoir, vial, syringe, or tubing to convey medication or fluid to or from a patient. Exemplary pumps include but are not limited to enteral pumps, infusion pumps, patient controlled analgesia (PCA) or pain management medication pumps, syringe pumps, peristaltic pumps and the like. In one embodiment, the infusion pump with multiple drug library editor source capability can be an infusion pump as described below in association with **FIG. 4****.**

Exemplary medication management systems with standalone, dedicated drug library editors deployed on or used on a standalone local computer include the GemStar™ SP Infusion System from Hospira, Inc., of Lake Forest, Illinois.

**FIG. 4** is a block diagram of an infusion pump with multiple drug library editor source capability in accordance with the present invention. The infusion pump can receive an operational drug library from either one of a dedicated drug library editor or an enterprise drug library editor.

The infusion pump **400** is for selective use with either one of a first drug library editor or a second drug library editor as described above. The first drug library editor is operable to generate a first drug library and the second drug library editor is operable to generate a second drug library. One of the first drug library editor and the second drug library editor is a dedicated drug library editor operably connectable to and providing a drug library for a single infusion pump or a single type of infusion pump. The other of the first drug library editor and the second drug library editor is an enterprise drug library editor operably connectable to and providing drug libraries for a large number of infusion pumps or different types of infusion pumps.

The infusion pump **400** includes a memory **410** operable to store an operational drug library **412,** a flow controller **420** operably connected to the memory **410,** and a fluid driver **430** operably connected to the flow controller **420.** The memory **410** is operable to receive one of the first drug library from the first drug library editor and the second drug library from the second drug library editor as the operational drug library **412.** The flow controller **420** is operable to control the fluid driver **430** in accordance with the received operational drug library **412** and delivering the infusion to the patient **450.** In one embodiment, the infusion pump **400** delivers the infusion from a fluid reservoir **440** to the patient **450.** In one embodiment, the fluid driver **430** acts upon a fluid containing device, such as a cassette, reservoir, vial, syringe, tubing, or the like, to convey medication or fluid to or from a patient.

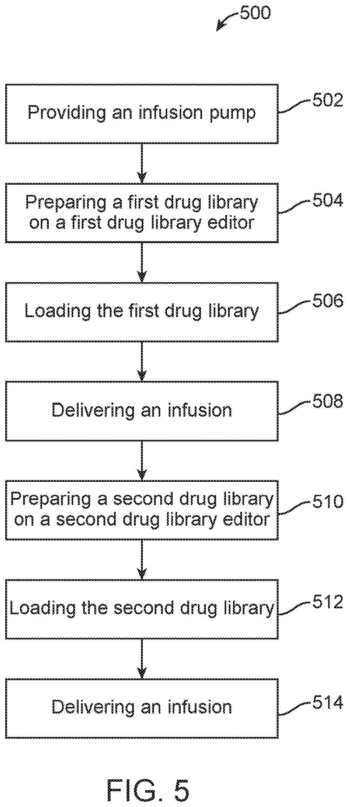

**FIG. 5** is a flowchart of a method of use for an infusion pump system with multiple drug library editor source capability in accordance with one embodiment of the present invention. The method can be used with the infusion pump system and infusion pump described above in conjunction with **FIGS. 1-4****.**

Referring to **FIG. 5****,** the method **500** includes providing an infusion pump **502** having an operational drug library; preparing a first drug library on a first drug library editor **504;** loading the first drug library **506** into the infusion pump from the first drug library editor as the operational drug library; delivering an infusion **508** from the infusion pump in accordance with the first drug library; preparing a second drug library on a second drug library editor **510;** loading the second drug library **512** into the infusion pump from the second drug library editor as the operational drug library; and delivering an infusion **514** from the infusion pump in accordance with the second drug library. The first drug library editor is one of a dedicated drug library editor and an enterprise drug library editor, and the second drug library editor is the other of the one of the dedicated drug library editor and the enterprise drug library editor.

In one embodiment, the first drug library editor is the dedicated drug library editor deployed on a local computer operably connectable to and local to the infusion pump. The local computer can be a personal computer, tablet computer, personal digital assistant, handheld computer, or the like. In one embodiment, the delivering an infusion **508** from the infusion pump in accordance with the first drug library further includes delivering the infusion from the infusion pump at a location such as a hospice, a nursing home, a home care environment, or other small treatment location.

In one embodiment, the infusion pump is one of a large number or multitude of infusion pumps or plurality of types of infusion pumps and the first drug library editor is the enterprise drug library editor deployed on a medication management unit operably connectable to and remote from the multitude of infusion pumps or plurality of types of infusion pumps. In one embodiment, the first drug library editor is the enterprise drug library editor deployed on a medication management unit operably connectable to and remote from the infusion pump, and the delivering an infusion **508** from the infusion pump in accordance with the first drug library further includes delivering the infusion from the infusion pump at a location such as a hospital, a treatment center, or other large treatment location.

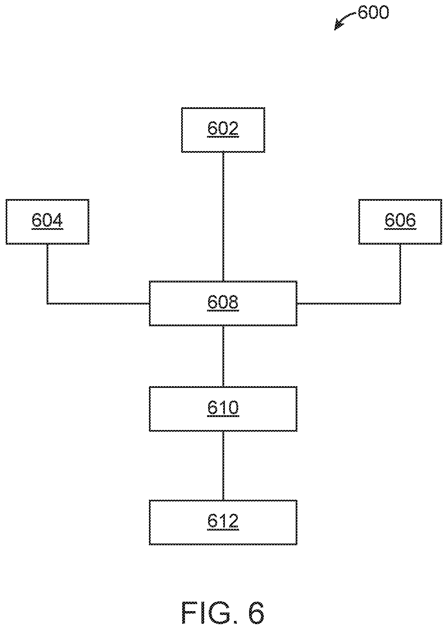

**FIG. 6** is a flowchart of another embodiment of a method of use for an infusion pump system with multiple drug library editor source capability in accordance with the present invention. The method can be used with the infusion pump system and infusion pump described above in conjunction with **FIGS. 1-4****.**

Referring to **FIG.** 6, the method **600** includes the step **602** of providing an infusion pump with multiple drug library editor source capability; preparing in step **604** a first drug library using a first drug library editor and/or preparing in step **606** a second drug library using a second drug library editor; selecting in step **608** a source drug library editor from one of the first drug library editor and the second drug library editor; loading in step **610** one of the first drug library and the second drug library into the infusion pump from the selected source drug library editor as an operational drug library; and in step **612** operating the infusion pump in accordance with the loaded operational drug library, in other words using the configuration and operational parameters defined in the operational drug library.

In one embodiment of the method **600** the step **608** of selecting a source drug library editor is performed at the infusion pump by a user making a selection from a plurality of possible selections on a user interface of the pump. In one embodiment, a download drug library function could be facilitated by a pump user interface display screen with a drop-down menu of drug library editor sources. The operational drug library is requested by the infusion pump to be downloaded from the selected source drug library editor. In another embodiment, the selecting of the source drug library editor is performed at one of the first and second drug library editors by a user of the drug library editors. A drug library editor user interface display screen can provide a drop-down menu or other means of selection for the drug library editor source. Then the selected drug library editor source can download the respective drug library to the target pump or pumps. In another embodiment, a special handshake between the drug library editor and the pump could identify the drug library editor source selected at the drug library editor. The download message or drug library itself could contain a code, configuration setting or direction as to where to accept the next drug library from. In yet another embodiment, the selection of the source drug library editor is performed at the medication management unit (MMU) depending on healthcare facility preferences and practices for a given infusion pump or set of infusion pumps.

One advantage of the present invention is that it provides a way to migrate one or more pumps from a dedicated drug library editor system to an enterprise drug library editor system without having to change out the pumps. It is also possible to remove pumps from an enterprise drug library editor environment and place them in a dedicated drug library environment like an alternative care site, nursing home, or patient's home without changing the pump. This seamless repurposing of pumps makes their inventory and associated capital costs easier to manage. Finally, the present invention allows the dedicated drug library editor to be more focused and therefore optimized in its limited functions for the type of pump it serves. The less complex dedicated drug library editor would be less costly to produce (and purchase), easier to setup, understand and run. Conversely, an enterprise drug library editor can be more easily life cycle managed, such as upgraded with bug fixes, new components or additional functionality, pump types, etc. The present invention allows for the leveraging of the advantages of both types of drug library editors in a single pump with multiple drug library editor capability.

While the embodiments of the invention disclosed herein are presently considered to be preferred, various changes, rearrangement of steps, and modifications can be made without departing from the scope of the invention. The scope of the invention is indicated in the appended claims, and all changes that come within the meaning and range of equivalents are intended to be embraced therein.

## Claims

1. An infusion pump (130, 240, 320, 400) for selectable use with either one of a first drug library editor (110) operable to generate a first drug library (112) and a second drug library editor (120) operable to generate a second drug library (122), the first drug library editor being one of a dedicated drug library editor (312) and an enterprise drug library editor (222), and the second drug library editor being the other of the one of the dedicated drug library editor and the enterprise drug library editor, wherein the dedicated drug library editor is connectable to and provides a drug library for a single infusion pump or a single type of infusion pump, and the enterprise drug library editor is connectable to and provides drug libraries for a plurality of infusion pumps or a plurality of types of infusion pumps, the infusion pump comprising:
a memory (410) operable to store an operational drug library (132, 242, 322, 412);
a flow controller (420) operably connected to the memory; and
a fluid driver (430) operably connected to the flow controller;
wherein the memory is operable to receive a selection of one of the first drug library from the first drug library editor and the second drug library from the second drug library editor as the operational drug library, the operational drug library is received from a source drug library editor of any of the first or second drug library editors; and
the flow controller is operable to control the fluid driver while the infusion pump is operated in accordance with configuration and operational parameters defined in the received operational drug library,
wherein a handshake between the infusion pump and any of the first and second drug library editors identifies said source drug library editor,
wherein the operational drug library further contains a configuration setting as to where to accept the next drug library from to migrate the infusion pump from one of the first and second drug library editors to the other one of the first or second drug library editors.

2. The infusion pump of claim 1 wherein the first drug library editor is the dedicated drug library editor deployed on a local computer operably connectable to and local to the infusion pump.

3. The infusion pump of claim 1 wherein the infusion pump is one type of a plurality of different types of infusion pumps and wherein the first drug library editor is the enterprise drug library editor deployed on a medication management unit (220) operably connectable to and remote from the plurality of different types of infusion pumps.

4. The infusion pump of claim 1 wherein the fluid driver is operable to act on a fluid containing device selected from the group consisting of a cassette, reservoir, vial, syringe, and tubing.

5. The infusion pump of claim 1 wherein a signal regarding the selection of one of the first drug library from the first drug library editor and the second drug library from the second drug library editor is generated at the infusion pump through user selection on a user interface of the infusion pump.

6. The infusion pump of claim 1 wherein a signal regarding the selection of one of the first drug library from the first drug library editor and the second drug library from the second drug library editor is generated through a setting in one of the first drug library editor and the second drug library editor.

7. A method (600) of configuring one or more infusion pumps comprising:
providing an infusion pump adapted to receive a drug library from one of multiple drug library editors and having an operational drug library;
preparing (604, 606) one of a first drug library on a first drug library editor or a second drug library on a second drug library editor;
selecting (608) one of the first drug library editor and the second drug library editor as a source drug library editor for a received drug library comprising one of the first drug library and the second drug library;
loading (610) the received drug library into the infusion pump as the operational drug library;
delivering (612) an infusion from the infusion pump in accordance with configuration and operational parameters defined in the operational drug library;
identifying the source drug library editor by a handshake between the infusion pump and any of the first and second drug library editors,
wherein the operational drug library further contains a configuration setting as to where to accept the next drug library from to migrate the infusion pump from one of the first and second drug library editors to the other one of the first or second drug library editors,
wherein the first drug library editor is one of a dedicated drug library editor and an enterprise drug library editor, and the second drug library editor is the other of the one of the dedicated drug library editor and the enterprise drug library editor, wherein the dedicated drug library editor is connectable to and provides a drug library for a single infusion pump or a single type of infusion pump, and the enterprise drug library editor is connectable to and provides drug libraries for a plurality of infusion pumps or a plurality of types of infusion pumps.

8. The method of claim 7 wherein the step of selecting one of the first drug library editor and the second drug library editor as a source for a received drug library is done at the infusion pump through user selection on a user interface of the infusion pump.

9. The method of claim 7 wherein the step of selecting of one of the first drug library editor and the second drug library editor as a source for a received drug library is accomplished through a setting in one of the first drug library editor and the second drug library editor.

## Patentansprüche

1. Infusionspumpe (130, 240, 320, 400) zur auswählbaren Verwendung mit einem von einem ersten Arzneimitteldatenbank-Editor (110), der betriebsfähig ist, eine erste Arzneimitteldatenbank (112) zu generieren, und einem zweiten Arzneimitteldatenbank-Editor (120), der betriebsfähig ist, eine zweite Arzneimitteldatenbank (122) zu generieren, wobei der erste Arzneimitteldatenbank-Editor einer von einem dedizierten Arzneimitteldatenbank-Editor (312) und einem Firmenarzneimitteldatenbank-Editor (222) ist, und der zweite Arzneimitteldatenbank-Editor der andere von dem dedizierten Arzneimitteldatenbank-Editor und dem Firmenarzneimitteldatenbank-Editor ist, wobei der dedizierte Arzneimitteldatenbank-Editor an eine einzige Infusionspumpe oder eine einzige Infusionspumpenart anschließbar ist und eine Arzneimitteldatenbank dafür bereitstellt, und der Firmenarzneimitteldatenbank-Editor an eine Vielzahl von Infusionspumpen oder eine Vielzahl von Infusionspumpenarten anschließbar ist und Arzneimitteldatenbanken dafür bereitstellt, wobei die Infusionspumpe umfasst:
einen Speicher (410), der betriebsfähig ist, eine Betriebsmedikamentendatenbank (132, 242, 322, 412) zu speichern;
einen Flussregler (420), der betriebsfähig an den Speicher angeschlossen ist; und
einen Fluidantrieb (430), der betriebsfähig an den Flussregler angeschlossen ist;
wobei der Speicher betriebsfähig ist, eine Auswahl von einer von der ersten Arzneimitteldatenbank von dem ersten Arzneimitteldatenbank-Editor und von der zweiten Arzneimitteldatenbank von dem zweiten Arzneimitteldatenbank-Editor als Betriebsmedikamentendatenbank zu empfangen, wobei die Betriebsmedikamentendatenbank von einem Quellmedikamentendatenbank-Editor von einem von den ersten oder zweiten Arzneimitteldatenbank-Editoren empfangen wird; und
der Flussregler betriebsfähig ist, den Fluidantrieb zu steuern, während die Infusionspumpe gemäß den Konfigurations- und Betriebsparametern betätigt wird, die in der empfangenen Betriebsmedikamentendatenbank definiert sind,
wobei ein Handshake zwischen der Infusionspumpe und einem von den ersten und zweiten Arzneimitteldatenbank-Editoren den Quellmedikamentendatenbank-Editor identifiziert,
wobei die Betriebsmedikamentendatenbank ferner eine Konfigurationseinstellung enthält, die angibt, woher die nächste Arzneimitteldatenbank anzunehmen ist, um die Infusionspumpe von einem von den ersten und zweiten Arzneimitteldatenbank-Editoren zu dem anderen von den ersten oder zweiten Arzneimitteldatenbank-Editoren umzustellen.

2. Infusionspumpe nach Anspruch 1, wobei der erste Arzneimitteldatenbank-Editor der dedizierte Arzneimitteldatenbank-Editor ist, der auf einem lokalen Computer installiert ist, der an die Infusionspumpe betriebsfähig anschließbar ist und an dieser lokal ist.

3. Infusionspumpe nach Anspruch 1, wobei die Infusionspumpe eine Art einer Vielzahl von verschiedenen Infusionspumpenarten ist, und wobei der erste Arzneimitteldatenbank-Editor der Firmenarzneimitteldatenbank-Editor ist, der auf einer Arzneimittelverwaltungseinheit (220) installiert ist, die an die Vielzahl von verschiedenen Infusionspumpenarten betriebsfähig anschließbar und von dieser entfernt ist.

4. Infusionspumpe nach Anspruch 1, wobei der Fluidantrieb betriebsfähig ist, um auf eine ein Fluid enthaltende Vorrichtung einzuwirken, die aus der Gruppe ausgewählt wird, die aus einer Kassette, einem Behälter, einem Injektionsfläschchen, einer Spritze und Schläuchen besteht.

5. Infusionspumpe nach Anspruch 1, wobei ein Signal bezüglich der Auswahl einer von der ersten Arzneimitteldatenbank von dem ersten Arzneimitteldatenbank-Editor und der zweiten Arzneimitteldatenbank von dem zweiten Arzneimitteldatenbank-Editor an der Infusionspumpe durch eine Benutzerauswahl an einer Benutzerschnittstelle der Infusionspumpe generiert wird.

6. Infusionspumpe nach Anspruch 1, wobei ein Signal bezüglich der Auswahl einer von der ersten Arzneimitteldatenbank von dem ersten Arzneimitteldatenbank-Editor und der zweiten Arzneimitteldatenbank von dem zweiten Arzneimitteldatenbank-Editor durch eine Einstellung in einem von dem ersten Arzneimitteldatenbank-Editor und dem zweiten Arzneimitteldatenbank-Editor generiert wird.

7. Verfahren (600) zum Konfigurieren einer oder mehrerer Infusionspumpen, umfassend folgende Schritte:
Bereitstellen einer Infusionspumpe, die geeignet ist, eine Arzneimitteldatenbank von einem von mehreren Arzneimitteldatenbank-Editoren zu empfangen, und eine Betriebsmedikamentendatenbank aufweist;
Vorbereiten (604, 606) einer von einer ersten Arzneimitteldatenbank in einem ersten Arzneimitteldatenbank-Editor oder einer zweiten Arzneimitteldatenbank in einem zweiten Arzneimitteldatenbank-Editor;
Auswählen (608) eines von dem ersten Arzneimitteldatenbank-Editor und dem zweiten Arzneimitteldatenbank-Editor als einen Quellmedikamentendatenbank-Editor für eine empfangene Arzneimitteldatenbank, die eine von der ersten Arzneimitteldatenbank und der zweiten Arzneimitteldatenbank umfasst;
Laden (610) der empfangenen Arzneimitteldatenbank in die Infusionspumpe als Betriebsmedikamentendatenbank;
Abgeben (612) einer Infusion aus der Infusionspumpe gemäß Konfigurations- und Betriebsparametern, die in der die Betriebsmedikamentendatenbank definiert sind;
Identifizieren des Quellmedikamentendatenbank-Editors durch einen Handshake zwischen der Infusionspumpe und einem von den ersten und zweiten Arzneimitteldatenbank-Editoren,
wobei die Betriebsmedikamentendatenbank ferner eine Konfigurationseinstellung enthält, die angibt, woher die nächste Arzneimitteldatenbank anzunehmen ist, um die Infusionspumpe von einem von den ersten und zweiten Arzneimitteldatenbank-Editoren auf den anderen von den ersten oder zweiten Arzneimitteldatenbank-Editoren umzustellen,
wobei der erste Arzneimitteldatenbank-Editor einer von einem dedizierten Arzneimitteldatenbank-Editor und einem Firmenarzneimitteldatenbank-Editor ist, und der zweite Arzneimitteldatenbank-Editor der andere von dem dedizierten Arzneimitteldatenbank-Editor und dem Firmenarzneimitteldatenbank-Editor ist, wobei der dedizierte Arzneimitteldatenbank-Editor an eine einzige Infusionspumpe oder eine einzige Infusionspumpenart anschließbar ist und eine Arzneimitteldatenbank dafür bereitstellt, und der Firmenarzneimitteldatenbank-Editor an eine Vielzahl von Infusionspumpen oder eine Vielzahl von Infusionspumpenarten anschließbar ist und Arzneimitteldatenbanken dafür bereitstellt.

8. Verfahren nach Anspruch 7, wobei der Schritt des Auswählens eines von dem ersten Arzneimitteldatenbank-Editor und dem zweiten Arzneimitteldatenbank-Editor als eine Quelle für eine empfangene Arzneimitteldatenbank an der Infusionspumpe über eine Benutzerauswahl an einer Benutzerschnittstelle der Infusionspumpe erfolgt.

9. Verfahren nach Anspruch 7, wobei der Schritt des Auswählens eines von dem ersten Arzneimitteldatenbank-Editor und dem zweiten Arzneimitteldatenbank-Editor als eine Quelle für eine empfangene Arzneimitteldatenbank durch eine Einstellung in einem von dem ersten Arzneimitteldatenbank-Editor und dem zweiten Arzneimitteldatenbank-Editor erreicht wird.

## Revendications

1. Pompe à perfusion (130, 240, 320, 400) pour une utilisation sélectionnable avec l'un ou l'autre d'un premier éditeur de pharmacothèque (110) pouvant fonctionner pour générer une première pharmacothèque (112) et d'un second éditeur de pharmacothèque (120) pouvant fonctionner pour générer une seconde pharmacothèque (122), le premier éditeur de pharmacothèque étant un certain parmi un éditeur de pharmacothèque dédié (312) et d'un éditeur de pharmacothèque d'entreprise (222), et le second éditeur de pharmacothèque étant l'autre du certain parmi l'éditeur de pharmacothèque dédié et l'éditeur de pharmacothèque d'entreprise, dans laquelle l'éditeur de pharmacothèque dédié peut être connecté à et fournit une pharmacothèque pour une pompe à perfusion unique ou un type de pompe à perfusion unique, et l'éditeur de pharmacothèque d'entreprise peut être connecté à et fournit des pharmacothèques pour une pluralité de pompes à perfusion ou une pluralité de types de pompes à perfusion, la pompe à perfusion comprenant :
une mémoire (410) pouvant fonctionner pour stocker une pharmacothèque opérationnelle (132, 242, 322, 412) ;
un dispositif de commande d'écoulement (420) connecté de manière opérationnelle à la mémoire ; et
un pilote de fluide (430) connecté de manière opérationnelle au dispositif de commande d'écoulement ;
dans laquelle la mémoire peut fonctionner pour recevoir une sélection de l'une de la première pharmacothèque à partir du premier éditeur de pharmacothèque et de la seconde pharmacothèque à partir du second éditeur de pharmacothèque en tant que pharmacothèque opérationnelle, la pharmacothèque opérationnelle est reçue d'un éditeur de pharmacothèque source de l'un quelconque des premier ou second éditeurs de pharmacothèque ; et
le dispositif de commande d'écoulement peut fonctionner pour commander le pilote de fluide pendant que la pompe à perfusion fonctionne conformément à une configuration et des paramètres de fonctionnement définis dans la pharmacothèque opérationnelle reçue,
dans laquelle un établissement de liaison entre la pompe à perfusion et l'un quelconque des premier et second éditeurs de pharmacothèque identifie ledit éditeur de pharmacothèque source,
dans laquelle la pharmacothèque opérationnelle contient en outre un réglage de configuration indiquant où accepter la pharmacothèque suivante pour faire migrer la pompe à perfusion de l'un des premier et second éditeurs de pharmacothèque vers l'autre des premier ou second éditeurs de pharmacothèque.

2. Pompe à perfusion selon la revendication 1, dans laquelle le premier éditeur de pharmacothèque est l'éditeur de pharmacothèque dédié déployé sur un ordinateur local pouvant être connecté de manière opérationnelle à la pompe à perfusion, et local par rapport à celle-ci.

3. Pompe à perfusion selon la revendication 1, dans laquelle la pompe à perfusion est un type d'une pluralité de types différents de pompes à perfusion et dans laquelle le premier éditeur de pharmacothèque est l'éditeur de pharmacothèque d'entreprise déployé sur une unité de gestion de médicaments (220) pouvant être connectée de manière fonctionnelle à et éloignée de la pluralité de différents types de pompes à perfusion.

4. Pompe à perfusion selon la revendication 1, dans laquelle le dispositif d'entraînement de fluide peut fonctionner pour agir sur un dispositif contenant un fluide choisi dans le groupe comprenant une cassette, un réservoir, un flacon, une seringue et une tubulure.

5. Pompe à perfusion selon la revendication 1, dans laquelle un signal concernant la sélection de l'une de la première pharmacothèque à partir du premier éditeur de pharmacothèque et de la seconde pharmacothèque à partir du second éditeur de pharmacothèque est généré au niveau de la pompe à perfusion par une sélection d'utilisateur sur une interface d'utilisateur de la pompe à perfusion.

6. Pompe à perfusion selon la revendication 1, dans laquelle un signal concernant la sélection de l'une de la première pharmacothèque à partir du premier éditeur de pharmacothèque et de la seconde pharmacothèque à partir du second éditeur de pharmacothèque est généré par un réglage dans l'un du premier éditeur de pharmacothèque et du second éditeur de pharmacothèque.

7. Procédé (600) de configuration d'une ou plusieurs pompes à perfusion comprenant les étapes consistant à :
fournir une pompe à perfusion adaptée pour recevoir une pharmacothèque à partir d'un de multiples éditeurs de pharmacothèque et ayant une pharmacothèque opérationnelle ;
préparer (604, 606) l'une d'une première pharmacothèque sur un premier éditeur de pharmacothèque ou d'une seconde pharmacothèque sur un second éditeur de pharmacothèque ;
sélectionner (608) l'un du premier éditeur de pharmacothèque et du second éditeur de pharmacothèque en tant qu'éditeur de pharmacothèque source pour une pharmacothèque reçue comprenant l'une de la première pharmacothèque et de la seconde pharmacothèque ;
charger (610) la pharmacothèque reçue dans la pompe à perfusion en tant que pharmacothèque opérationnelle ;
délivrer (612) une perfusion à partir de la pompe à perfusion conformément à la configuration et aux paramètres opérationnels définis dans la pharmacothèque opérationnelle ;
identifier l'éditeur de pharmacothèque source par un établissement d'une liaison entre la pompe à perfusion et l'un quelconque des premier et second éditeurs de pharmacothèque,
dans lequel la pharmacothèque opérationnelle contient en outre un réglage de configuration indiquant où accepter la pharmacothèque suivante pour faire migrer la pompe à perfusion de l'un des premier et second éditeurs de pharmacothèque vers l'autre des premier ou second éditeurs de pharmacothèque,
dans lequel le premier éditeur de pharmacothèque est l'un d'un éditeur de pharmacothèque dédié et d'un éditeur de pharmacothèque d'entreprise, et le second éditeur de pharmacothèque est l'autre de l'éditeur de pharmacothèque dédié et de l'éditeur de pharmacothèque d'entreprise, dans lequel l'éditeur de pharmacothèque dédié peut être connecté à et fournit une pharmacothèque pour une pompe à perfusion unique ou un type de pompe à perfusion unique, et l'éditeur de pharmacothèque d'entreprise peut être connecté à et fournit des pharmacothèques pour une pluralité de pompes à perfusion ou une pluralité de types de pompes à perfusion.

8. Procédé selon la revendication 7, dans lequel l'étape de sélection de l'un du premier éditeur de pharmacothèque et du second éditeur de pharmacothèque en tant que source pour une pharmacothèque reçue est effectuée au niveau de la pompe à perfusion par une sélection d'utilisateur sur une interface d'utilisateur de la pompe à perfusion.

9. Procédé selon la revendication 7, dans lequel l'étape de sélection de l'un du premier éditeur de pharmacothèque et du second éditeur de pharmacothèque en tant que source pour une pharmacothèque reçue est accomplie par un réglage dans l'un du premier éditeur de pharmacothèque et du second éditeur de pharmacothèque.
